# FASCICULE DE BREVET EUROPEEN

(11) **EP 3 573 544 B1**
(45) Date de publication et mention de la délivrance du brevet: **05.08.2020**
(21) Numéro de dépôt: 18713714.6
(22) Date de dépôt: 01.02.2018
(51) Int. Cl.: A61B 17/128, A61F 2/24, A61B 17/122, A61B 90/00

(54) **DISPOSITIF DE RÉPARATION DES CORDAGES DE LA VALVE MITRALE D'UN COEUR PAR VOIE TRANSFEMORALE**
VORRICHTUNG ZUR TRANSFEMORALEN REPARATUR DER MITRALKLAPPENSEHNENFÄDEN EINES HERZENS
DEVICE FOR TRANSFEMORAL REPAIR OF THE MITRAL VALVE CHORDAE OF A HEART

(30) Priorité: 13.03.2017 FR 1752019
(43) Date de publication de la demande: 04.12.2019
(73) Titulaire: CMI'NOV, 43120 Monistrol-sur-Loire (FR); Vola, Marco, 42270 Saint-Priest-en Jarez (FR)
(72) Inventeur: VOLA, Marco, 42270 Saint-Priest en Jarez (FR); PAIN, Bernard, 43120 Monistrol sur Loire (FR)
(74) Mandataire: Cabinet Laurent & Charras
(86) Numéro de dépôt international: PCT/FR2018/050243
(87) Numéro de publication internationale: WO 2018/167388

(56) Documents cités:
- WO-A1-2011/067770
- WO-A1-2013/112795
- WO-A2-2016/042022
- US-A1- 2016 345 959
- BAJONA P ET AL: "Beating-heart, off-pump mitral valve repair by implantation of artificial chordae tendineae: An acute in vivo animal study", JOURNAL OF THORACIC AND CARDIOVASCULAR SURGERY, MOSBY-YEAR BOOK, INC., ST. LOUIS, MO, US, vol. 137, no. 1, 1 janvier 2009 (2009-01-01), pages 188-193, XP025871792, ISSN: 0022-5223, DOI: 10.1016/J.JTCVS.2008.09.004 [extrait le 2009-01-09]

## Description

### DOMAINE TECHNIQUE

L'invention concerne un dispositif de réparation des cordages de la valve mitrale d'un cœur, notamment par voie transfémorale, par exemple en cas de prolapsus mitral.

### ETAT ANTERIEUR DE LA TECHNIQUE

Le traitement du prolapsus mitral, lié notamment à l'élongation ou la rupture d'un ou de plusieurs cordages reliant la valve mitrale aux muscles papillaires du cœur, est une opération longue et lourde qui nécessite l'ouverture des cavités cardiaques, de la cage thoracique et une circulation sanguine extracorporelle. L'opération consiste notamment à remplacer le cordage défectueux, c'est-à-dire à accrocher un cordage artificiel entre un feuillet de la valve mitrale et les piliers de l'appareil sous valvulaire mitral ou la partie apicale du cœur.

Une solution connue de l'état de la technique consiste à utiliser des dispositifs qui peuvent implanter des cordages artificiels sur les bords des valves balantes par la voie transapicale, c'est-à-dire à utiliser un dispositif destiné être positionné dans un introducteur étanche disposé dans la cavité thoracique entre deux côtes pour pénétrer dans le ventricule gauche en passant par l'apex du cœur.

Ce dispositif peut encore être amélioré, notamment en évitant de percer l'apex cardiaque et en diminuant l'invasivité de l'opération chirurgicale réalisée avec ce type de dispositifs. Le brevet WO 2016/042022 divulgue un dispositif de réparation d'un cordage de la valve mitrale d'un coeur par voie transfémorale. Le dispositif comprend un ancrage (10) de feuillet destiné à être placé dans un feuillet (12) d'une valvule cardiaque, l'ancrage (10) de feuillet étant agencé pour être accouplé à la corde artificielle (14) ; et un dispositif de préhension (6) mécanique pour saisir le feuillet (12) de la valvule cardiaque, le dispositif de préhension (6) comprenant un canal d'ancrage de feuillet pour loger l'ancrage (10) de feuillet dans une configuration pliée ; le dispositif de préhension (6) et l'ancrage (10) de feuillet étant agencés de telle sorte que, lors de l'utilisation, le dispositif de préhension (6) saisisse le feuillet (12), l'ancrage (10) de feuillet puisse être poussé hors du canal d'ancrage de feuillet pour percer le feuillet (12) et donner à l'ancrage (10) de feuillet une configuration dépliée de sorte que des formations en crochet de l'ancrage (10) de feuillet puissent, lors de l'utilisation, fixer l'ancrage (10) de feuillet dans le feuillet (12).

### EXPOSE DE L'INVENTION

L'invention s'est fixée pour but de remédier aux inconvénients de l'état de la technique d'une manière sûre, simple, efficace et rationnelle.

Le problème que se propose de résoudre l'invention est de faciliter l'opération de réparation des cordages de la valve mitrale d'un cœur, par voie transfémorale et sans ponctionner l'artère, mais la veine fémorale

A cet effet, il a été mis au point un dispositif de réparation des cordages de la valve mitrale d'un cœur par voie transfémorale, et destiné à être positionné dans un introducteur étanche disposé dans une veine fémorale pour pénétrer dans l'oreillette gauche du cœur en passant par la paroi du septum qui sépare l'oreillette droite et gauche.

Selon l'invention, le dispositif comprend :
- un premier ensemble apte à coopérer avec une poignée assujettie à des moyens de commande pour l'actionnement de l'ensemble pour la mise en place et la fixation d'un cordage artificiel sur le pilier d'un cordage rompu ou pathologiquement élongé, ledit ensemble est agencé à l'extrémité d'une tige de manipulation et comprend une agrafe élastique reliée de manière pivotante et amovible à l'extrémité de la tige de sorte à passer, sous l'action des moyens de commande, d'une position repliée le long de la tige à une position basculée angulairement par rapport à la tige pour se placer perpendiculairement face au pilier où le cordage doit être remplacé, le premier ensemble comprend des moyens d'ouverture de l'agrafe pour, sous l'action des moyens de commande, agrafer le pilier du cordage rompu, (soit par simple compression de deux pors avec surface irrégulière, soit par compression des deux mors de l'agraphe lesquels contiennent des extrémités perforant le pilier, partiellement ou le transfixiant totalement),le premier ensemble comprend des moyens de déverrouillage permettant, sous l'action des moyens de commande, de libérer l'agrafe, ladite agrafe étant reliée à un cordage artificiel qui remonte dans la tige ;
- un deuxième ensemble apte à coopérer avec une poignée assujettie à des moyens de commande pour l'actionnement de l'ensemble pour la mise en place et la fixation du cordage artificiel à un feuillet de la valve, le deuxième ensemble est agencé à l'extrémité d'une tige de manipulation et comprend des moyens de fixation destinés à coulisser le long du cordage artificiel et reliés de manière pivotante à l'extrémité de la tige de sorte à passer, sous l'action des moyens de commande, d'une position repliée le long de la tige à une position basculée angulairement par rapport à la tige pour se placer parallèlement au feuillet mitral, les moyens de fixation étant aptes, sous l'action des moyens de commande, à fixer le cordage artificiel audit feuillet.

De cette manière, et d'une manière avantageuse, le dispositif selon l'invention permet, en combinaison avec une poignée et des moyens d'actionnement qui ne font pas partie de l'invention, de réaliser l'opération de réparation d'un cordage de la valve mitrale par la voie transfémorale en remontant par la veine cave. Les ensembles actifs du dispositif naviguent dans la veine cave où la pression sanguine est relativement faible.

Selon une première forme de réalisation, les moyens de fixation comprennent une agrafe élastique ouvrable, repositionnable et amovible, des moyens d'ouverture de l'agrafe, et des moyens de déverrouillage de l'agrafe similaires à ceux du premier ensemble. L'agrafe du deuxième ensemble est destinée à s'ancrer sur le feuillet pour y fixer le cordage artificiel

Le cordage artificiel se présente par exemple sous la forme d'un fil de suture non résorbable en polyamide ou téflon.

Le cordage artificiel peut être cranté, et l'agrafe du deuxième ensemble comprend des moyens pour coulisser sur le cordage artificiel cranté uniquement en direction de l'agrafe du premier ensemble.

Selon une autre forme de réalisation, l'agrafe du deuxième ensemble comprend un fil destiné à former une boucle de fixation autour du cordage artificiel, le cordage artificiel étant apte à être noué autour de ladite boucle.

Avantageusement, le deuxième ensemble comprend une pince stabilisatrice reliée de manière pivotante à l'extrémité de la tige de sorte à passer, sous l'action des moyens de commande, d'une position repliée le long de la tige à une position basculée angulairement à la tige pour se trouver parallèle au plan du feuillet, et d'une position ouverte à une position fermée pour pincer et stabiliser le feuillet en vue de l'agrafage.

Selon une autre forme de réalisation particulière, les moyens de fixation comprennent une pince reliée de manière pivotante à l'extrémité de la tige de sorte à passer, sous l'action des moyens de commande, d'une position repliée le long de la tige à une position basculée angulairement à la tige pour se trouver parallèle au plan du tissu du feuillet, et d'une position ouverte à une position fermée pour pincer le feuillet. Le deuxième ensemble comprend une aiguille mobile pour venir, sous l'action des moyens de commande, percer le feuillet au travers d'une ouverture que présente la pince et fixer le cordage artificiel au feuillet.

De préférence, l'aiguille comprend intérieurement une suture à mémoire de forme apte à être extraite pour former une boucle pour fixer le cordage artificiel au feuillet.

Selon une autre forme de réalisation particulière, l'aiguille comprend des moyens pour saisir le cordage artificiel après avoir percé le feuillet.

Selon une forme de réalisation particulière, l'agrafe comprend deux branches élastiques rapprochées l'une de l'autre avec des parties d'extrémités en crochet. Les moyens d'ouverture/déverrouillage comprennent deux bras disposés entre les branches élastiques de l'agrafe et articulés autour d'un pivot de sorte à passer, sous l'action des moyens de commande, d'une position rapprochée l'un de l'autre à une position écartée en forçant l'écartement des branches de l'agrafe pour provoquer son ouverture. Les deux bras sont escamotables pour permettre la libération de l'agrafe amovible.

De préférence, la pince comprend un mors supérieur et un mors inférieur, ajourés pour le passage de l'aiguille. Le mors inférieur comprend un clapet destiné à adopter une position fermée pour maintenir le cordage artificiel, tout en autorisant le coulissement de la pince autour et le long dudit cordage artificiel, et une position ouverte de libération du cordage artificiel.

De préférence, et pour faciliter l'opération chirurgicale, l'agrafe et les moyens de fixation sont radio-opaques ou bien comprennent chacun un ou plusieurs marqueur radio-opaque et/ou échographique. Ils peuvent également incorporer des magnets pour faciliter la navigation sous la présence d'un champ magnétique spécifiquement généré.

Avantageusement, et pour faciliter la réhabilitation des tissues humains, la ou les agrafes sont gainées de textile.

### BREVE DESCRIPTION DES DESSINS

D'autres caractéristiques et avantages de l'invention ressortiront clairement de la description qui en est réalisée ci-après, à titre indicatif et nullement limitatif, en référence aux figures schématiques annexées dans lesquelles :
- la figure 1 illustre un cœur et l'introduction, dans l'oreillette gauche du cœur et au travers de la valve mitrale, de la tige de manipulation d'un premier ensemble que comprend le dispositif selon l'invention ;
- la figure 2 illustre est une vue similaire à celle de la figure 1, la tête de la tige étant escamotée pour laisser apparaître une agrafe élastique en position repliée ;
- la figure 3 est une vue similaire à celle de la figure 2, l'agrafe étant basculée dans une position orthogonale à la tige ;
- la figure 4 est une vue similaire à celle de la figure 3, l'agrafe étant venue s'ancrer sur le pilier du cordage rompu à réparer ;
- la figure 5 est une vue similaire à celle de la figure 4, la tige du premier ensemble ayant été retirée de manière à laisser un cordage artificiel relié à l'agrafe ;
- la figure 6 est une vue de détail de l'extrémité de la tige du premier ensemble selon l'invention, avec la tête escamotée et l'agrafe en position repliée ;
- la figure 7 est une vue similaire à celle de la figure 6, avec l'agrafe basculée dans une position orthogonale à la tige, et en position fermée ;
- la figure 8 est une vue similaire à celle de la figure 7, l'agrafe étant représentée en position ouverte ;
- la figure 9 est une vue de détail illustrant en perspective l'agrafe utilisée pour l'ancrage au pilier du cordage ;
- la figure 10 est une vue similaire à celle de la figure 9, l'agrafe étant représentée en position ouverte ;
- la figure 11 est une représentation d'un premier mode de réalisation pour l'attache du cordage artificiel au feuillet de la valve mitrale, et illustrant la tige de manipulation d'un deuxième ensemble ayant traversé la valve mitrale, la tête de la tige ayant été escamotée pour laisser apparaître une deuxième agrafe en position repliée le long de la tige ;
- la figure 12 est une vue similaire à celle de la figure 11, l'agrafe ayant été basculée dans une position orthogonale à la tige, l'agrafe ayant coulissé le long du cordage artificiel ;
- la figure 13 illustre l'ancrage de l'agrafe au feuillet de la valve mitrale, et la libération de ladite agrafe, laquelle est reliée au cordage artificiel ;
- la figure 14 est une vue similaire à celle de la figure 13, le cordage ayant été noué autour d'une boucle que comprend la deuxième agrafe, l'opération de remplacement du cordage étant terminée ;
- la figure 15 est une vue illustrant en perspective l'extrémité de la tige de manipulation du deuxième ensemble, avec l'agrafe en position basculée et ouverte ;
- la figure 16 est une représentation schématique d'un deuxième mode de réalisation, comprenant une tige d'extrémité d'un deuxième ensemble ayant traversé la valve mitrale du cœur, et illustrant une pince basculée en position angulaire par rapport à la tige et ayant coulissé le long du cordage artificiel relié à la première agrafe ;
- la figure 17 est une vue similaire à celle de la figure 16, la pince ayant pincé le feuillet de la valve mitrale ;
- la figure 18 est une vue similaire à celle de la figure 17, une suture ayant été libérée pour former une boucle permettant de fixer le cordage artificiel au feuillet ;
- la figure 19 est une vue similaire à celle de la figure 18, la tige de manipulation du deuxième ensemble étant représentée en train de se retirer ;
- la figure 20 est une vue similaire à celle de la figure 19, illustrant le cordage artificiel fixé au pilier du cordage et au feuillet de la valve par l'intermédiaire de la suture, avant l'étape consistant à nouer et mettre en tension ledit cordage artificiel pour terminer l'opération ;
- la figure 21 est une représentation schématique en perspective de la pince du deuxième ensemble selon le deuxième mode de réalisation, destiné à attacher le cordage artificiel au feuillet de la valve ;
- la figure 22 est une représentation schématique de la tige de manipulation du deuxième ensemble selon le deuxième mode de réalisation, la pince étant représentée en position repliée le long de la tige ;
- la figure 23 est une vue similaire à celle de la figure 22, la pince ayant été basculée angulairement par rapport à la tige et représentée en position ouverte, notamment avec le mors inférieur de la pince positionné sous le feuillet de la valve mitrale ;
- la figure 24 est une représentation schématique similaire à celle de la figure 23, la pince étant représentée en position fermée et en position de pincement du feuillet de la valve ;
- la figure 25 est une vue similaire à celle de la figure 24, une aiguille ayant été déplacée pour percer le feuillet de la valve mitrale ;
- la figure 26 est une vue similaire à celle de la figure 25, une suture ayant été extraite pour former une boucle autour du cordage artificiel et au travers du feuillet ;
- la figure 27 est une vue similaire à celle de la figure 26, l'aiguille ayant été retirée et le cordage artificiel ayant été libéré par la pince ;
- la figure 28 est une vue similaire à celle de la figure 27, la tige de manipulation du deuxième ensemble ayant été retirée, et le cordage artificiel étant fixé au feuillet par l'intermédiaire de la suture à mémoire de forme.

### EXPOSE DETAILLE DE L'INVENTION

L'invention concerne un dispositif (1) permettant de réaliser une opération chirurgicale consistant à réparer des cordages rompus ou élongés d'une valve mitrale (2), par exemple dans le cas d'un prolapsus mitral.

Le dispositif (1) est destiné à être positionné dans un introducteur étanche de tout type connu et approprié (non représenté) et d'un diamètre inférieur ou égal à 12 mm, disposé dans une veine fémorale pour remonter et pénétrer dans l'oreillette gauche (3) d'un cœur (4) en passant par sa paroi septale.

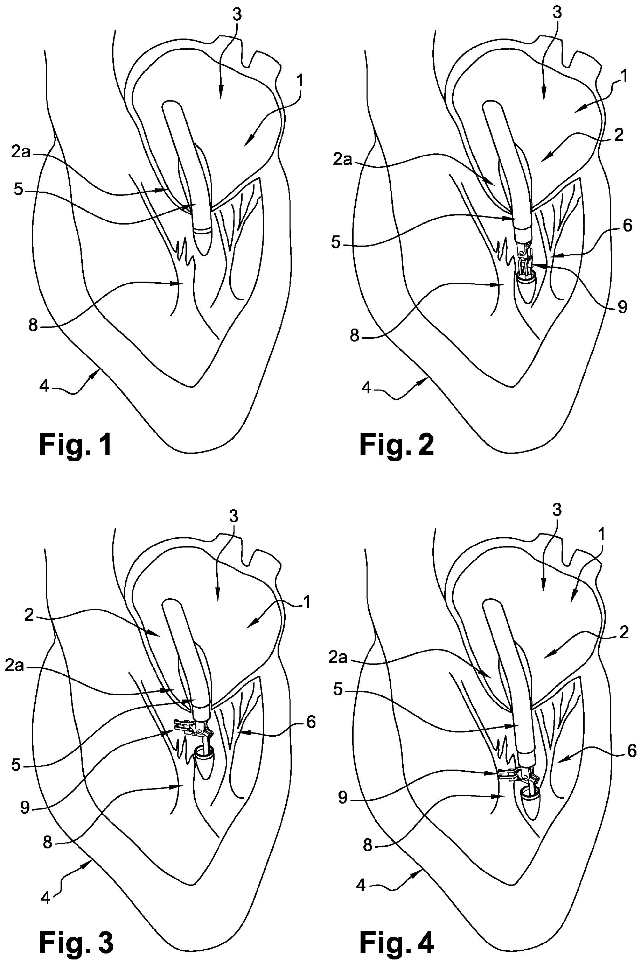

En référence aux figures 1 à 8, le dispositif (1) comprend une première tige de manipulation (5), à l'extrémité de laquelle est agencé un premier ensemble (6) pour la mise en place et la fixation d'un cordage artificiel (7) sur le pilier (8) d'un cordage rompu. L'autre extrémité de la première tige (5) est destinée à coopérer avec une poignée assujettie à des moyens de commande, non représentés, par exemple sous la forme d'une gâchette pour l'actionnement du premier ensemble (6). Le dispositif (1) selon l'invention permet, sous contrôle échographique et éventuellement aussi radiographique, après avoir remonté la veine cave, de faire pénétrer le premier ensemble (6) dans l'oreillette gauche (3) du cœur (4) en passant au travers de la paroi septale. Ensuite, lorsque le premier ensemble (6) se trouve dans l'oreillette gauche (3) du cœur (4), il est apte à passer au travers de la valve mitrale (2) pour venir en regard du pilier (8) du cordage rompu (Cf. figure 2). Le cathéter porteur peut être rigidifié temporairement avec la courbure idéale pour travailler en regard du plan de la valve mitrale.

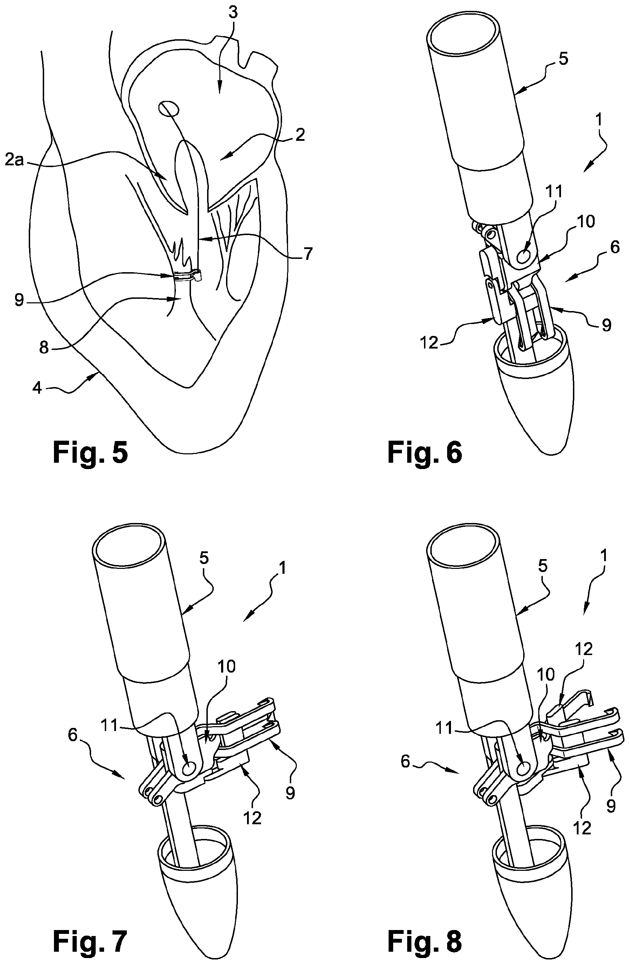

La tête de la première tige (5), constituée par exemple par une ogive atraumatique, est escamotable pour laisser apparaître intérieurement une agrafe élastique (9) reliée de manière pivotante et amovible à l'extrémité de la tige (5). L'agrafe élastique (9), assujettie au moyen de commande de la poignée, est apte à passer, sous l'action desdits moyens de commande, d'une position repliée le long la tige (Cf. figures 2 et 6) à une position basculée orthogonalement (Cf. figures 3, 4, 7, 8). À cet effet, l'agrafe élastique (9) est reçue dans un socle (10), lui-même monté pivotant autour d'un axe (11), sous l'actionnement des moyens de commande. Ces moyens de commande, non représentés, comprennent par exemple des jeux de câbles, reliés à une partie du socle (10), opposée à l'agrafe (9), pour effectuer une traction ou une pression sur cette partie arrière et faire pivoter le socle (10) autour de son axe (11).

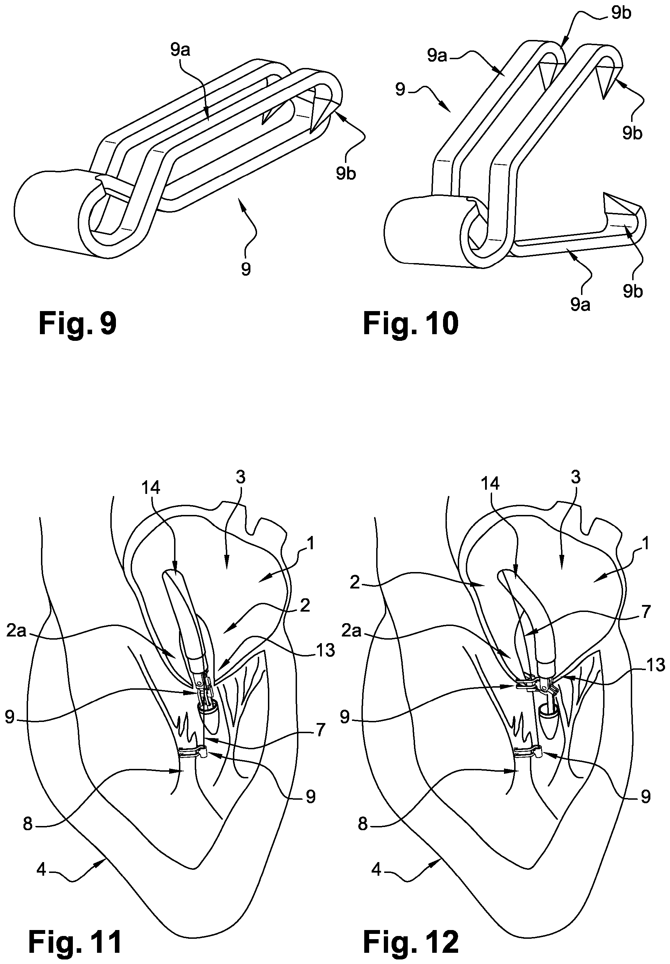

En référence aux figures 9 et 10, l'agrafe (9) comprend deux branches élastiques (9a), rapprochées l'une de l'autre, avec des parties d'extrémité en crochet (9b) pour l'ancrage en tant que tel. En référence aux figures 6 à 8, l'agrafe (9) est maintenue dans le socle (10) par l'intermédiaire de deux bras (12) disposés entre les branches élastiques (9a) de l'agrafe (9), et articulés autour d'un pivot, notamment autour de l'axe (11) de sorte à passer, sous l'action des moyens de commande, d'une position rapprochée l'un de l'autre (Cf. figures 6, 7, 15) à une position écartée en forçant l'écartement des branches (9a) de l'agrafe (9) pour provoquer son ouverture. Le pivotement des bras (12) est réalisé par le pivotement du socle (10).

L'accrochage de l'agrafe (9) sur le pilier (8) du cordage rompu (Cf. figure 4) permet de maintenir un écart suffisant entre les branches élastiques (9a) de l'agrafe (9), créant un jeu par rapport aux bras (12) qui constituent des moyens d'ouverture et de déverrouillage de l'agrafe (9), permettant auxdits bras (12) de s'escamoter et de libérer l'agrafe (9) amovible (Cf. figure 5).

En référence à la figure 5, le cordage artificiel (7) est noué à l'agrafe (9) et remonte à l'intérieur de la première tige (5) de sorte qu'après retrait de ladite tige, le cordage artificiel (7) remonte le long de la veine fémorale.

En référence aux figures 12 à 28, le dispositif (1) selon l'invention comprend également un deuxième ensemble (13) apte à coopérer avec une poignée, qui peut être la même poignée que celle du premier ensemble (6), assujettie à des moyens de commande pour l'actionnement de ce deuxième ensemble (13) pour la mise en place et la fixation du cordage artificiel (7) à un feuillet (2a) de la valve (2).

De la même manière que pour le premier ensemble (6), le deuxième ensemble (13) est destiné à remonter la veine cave, et à pénétrer dans l'oreillette gauche (3) du cœur (4) en passant au travers de la paroi septale, notamment en suivant et en coulissant le long du cordage artificiel (7) déjà présent.

Le deuxième ensemble (13) est agencé à l'extrémité d'une deuxième tige de manipulation (14) et comprend des moyens de fixation (9, 16), reliés de manière pivotante à l'extrémité de la tige de sorte à passer, sous l'action des moyens de commande, d'une position repliée le long de la tige (14) (Cf. figure 11) à une position basculée (Cf. figures 12, 15 à 18, 21 et 23 à 27) pour fixer le cordage artificiel (7) au feuillet (2a) de la valve (2).

À partir de ce concept, plusieurs modes de réalisation de l'invention ont été imaginés.

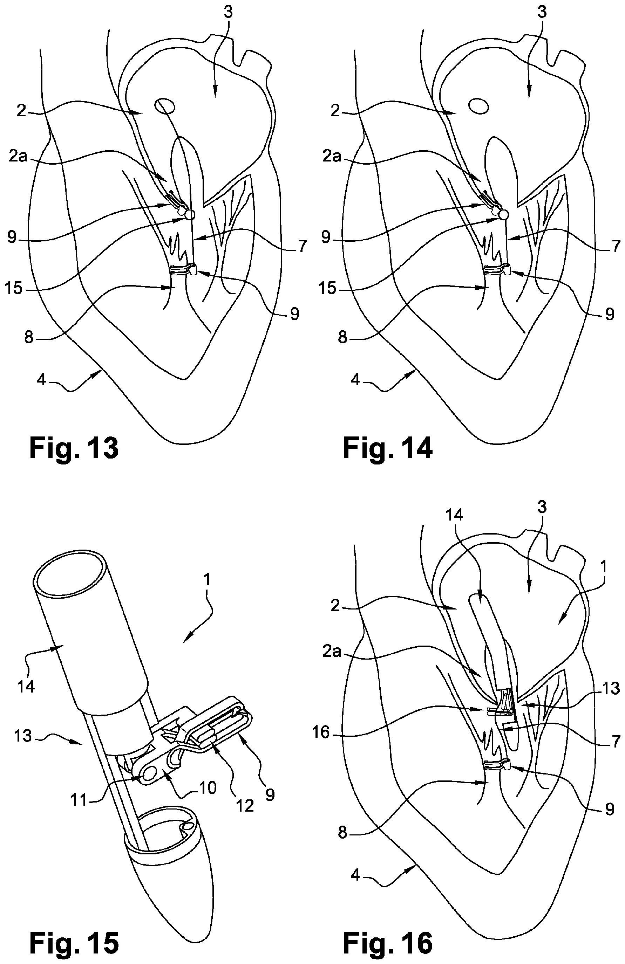

Selon un premier mode de réalisation, notamment illustré aux figures 11 à 15, les moyens de fixation du cordage artificiel (7) au feuillet (2a) se présentent sous la forme d'une agrafe (9), similaire à celle du premier ensemble (6), et qui est destinée à s'ancrer sur le pilier (8) du cordage rompu. De la même manière, cette deuxième agrafe (9) est reçue dans un socle (10) monté pivotant autour d'un axe (11) pour passer d'une position repliée le long de la deuxième tige (14) (Cf. figure 11) à une position basculée angulairement (Cf. figure 12, 15) afin de venir s'ancrer sur le feuillet (2a) de la valve mitrale (2). Des bras (12) sont disposés entre les branches élastiques (9a) de cette deuxième agrafe (9), pour constituer des moyens de verrouillage déverrouillage, de la même manière qu'avec la première agrafe (9). La deuxième agrafe (9) est donc conçue pour s'ouvrir et venir s'ancrer sur le feuillet (2a). Lorsque l'agrafe (9) est ancrée sur le feuillet (2a), les bras (12) peuvent s'escamoter pour libérer ladite agrafe (9).

La deuxième agrafe (9) est reliée au cordage artificiel (7). Pour ce faire, et en référence au figures 13 et 14, la deuxième agrafe (9) comprend, par exemple, un fil (15) qui forme une boucle autour du cordage artificiel (7). Cette boucle permet notamment à ladite deuxième agrafe (9) et au deuxième ensemble (13) de coulisser le long du cordage artificiel (7). Dans cette configuration, le cordage artificiel (7) peut être noué autour de la boucle et tendu pour terminer l'opération de réparation du cordage. Le cordage artificiel (7) est donc fixé au pilier (8) et au feuillet (2a) par l'intermédiaire d'agrafes (9).

Selon un deuxième exemple non illustré, le cordage artificiel (7), qui se présente par exemple sous la forme d'un fil de suture non résorbable en polyamide ou en téflon, peut-être cranté. Dans cette configuration, l'agrafe (9) du deuxième ensemble (13) peut comprendre tout moyen approprié, tel qu'une crémaillère, pour coulisser le long dudit cordage artificiel (7) cranté et uniquement en direction de l'agrafe (9) du premier ensemble (6). En d'autres termes, lorsque la deuxième agrafe (9) est ancrée sur le feuillet (2a), la tension du cordage artificiel (7) est ajustée en faisant coulisser la deuxième agrafe (9) le long du cordage artificiel (7) cranté.

L'ancrage de la deuxième agrafe (9) sur le feuillet (2a) de la valve (2) peut être facilité par l'intermédiaire d'une pince stabilisatrice, non représentée, similaire à la pince (16) qui sera décrite plus bas. À cet effet le deuxième ensemble (13) comprend une telle pince reliée de manière pivotante à l'extrémité de la deuxième tige (14) de sorte à passer, sous l'action des moyens de commande, d'une position repliée le long de la tige (14) à une position basculée régulièrement à la tige (14), c'est-à-dire parallèlement au feuillet (2a) de la valve (2), et d'une position ouverte à une position fermée pour pincer et stabiliser le feuillet (2a) en vue de l'ancrage de la deuxième agrafe élastique (9).

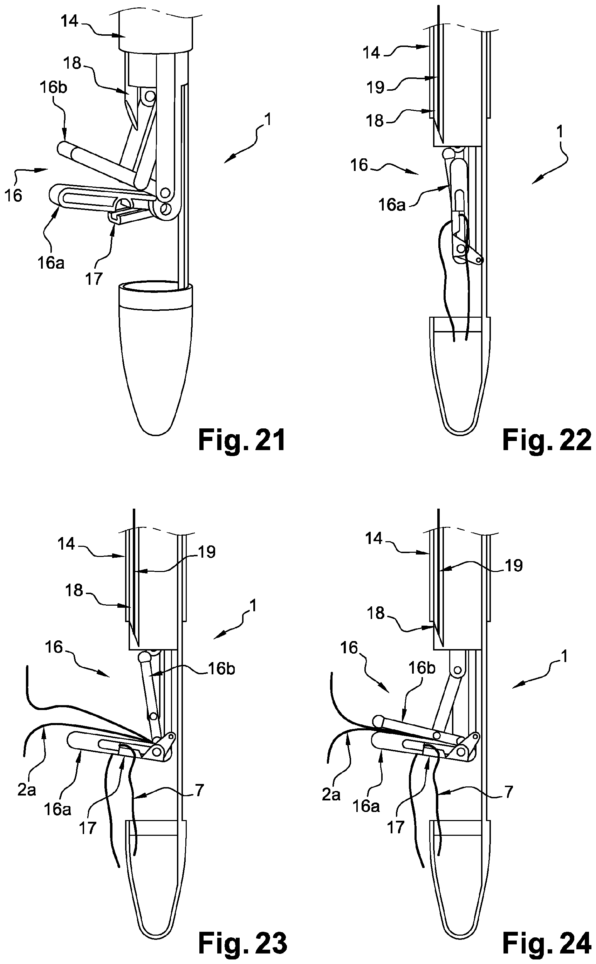

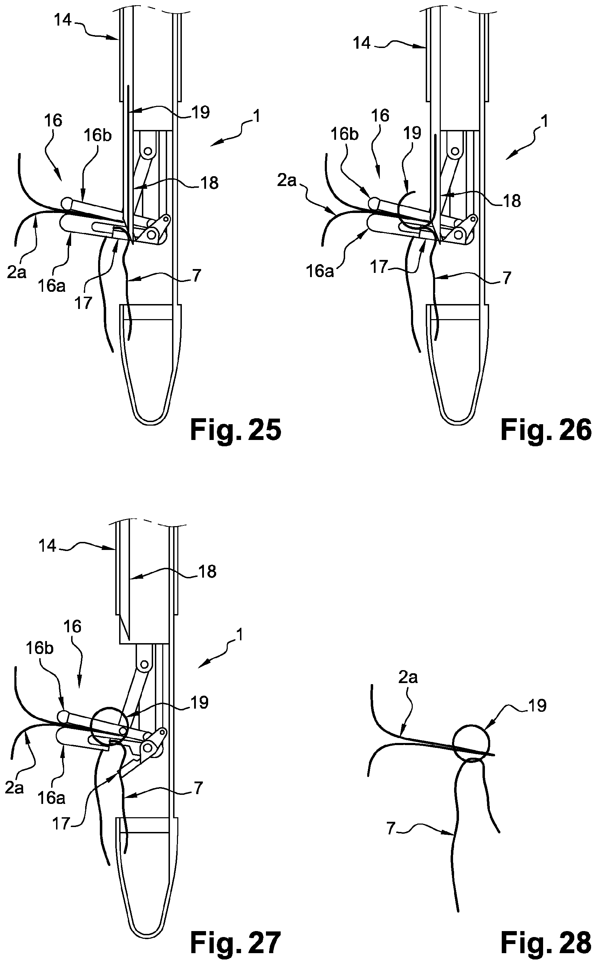

La fixation du cordage artificiel (7) au feuillet (2a) de la valve (2) peut être réalisée d'une manière différente. En effet, en référence aux figures 16 à 28, en lieu et place d'une deuxième agrafe (9), les moyens de fixation du deuxième ensemble (13) peuvent comprendre une pince (16) reliée de manière pivotante à l'extrémité de la deuxième tige (14) de sorte à passer, sous l'action des moyens de commande, d'une position repliée le long de la tige (14) (Cf. figure 22) à une position basculée angulairement (Cf. figures 16 à 18, 21 et 23 à 27), notamment parallèlement au feuillet (2a) de la valve (2). Cette pince (16) est conçue pour passer d'une position ouverte à une position fermée pour pincer le feuillet (2a), par l'action, par exemple, de câbles de poussée et/ou de poussée. La pince (16) est reliée au cordage artificiel (7) pour coulisser le long de celui-ci lors de l'introduction du deuxième ensemble (13) dans l'oreillette gauche (3) du cœur (4). À cet effet, la pince (16) comprend un mors supérieur (16b), et un mors inférieur (16a) équipé d'un clapet (17) articulé pour adopter une position fermée (Cf. figures 22 à 26) dans laquelle il enserre et maintient le cordage artificiel (7), tout en autorisant le coulissement de la pince (16) autour et le long dudit cordage artificiel (7), et une position ouverte (Cf. figures 21 et 27) de libération du cordage artificiel (7).

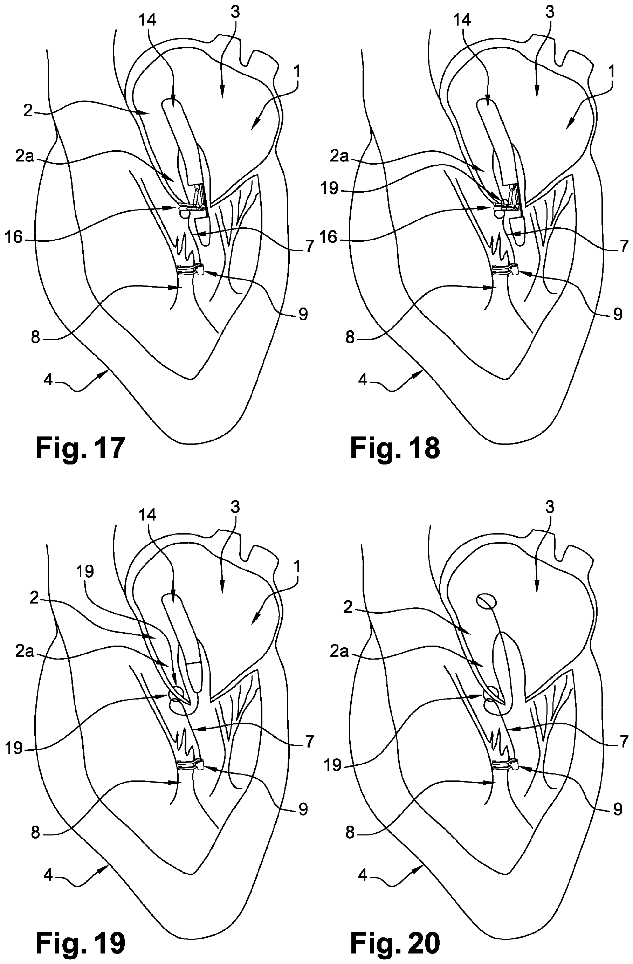

Les figures 21 à 28 illustrent les différentes étapes mises en oeuvre par le dispositif dans les étapes illustrées aux figures 16 à 20. En référence à ces figures, la pince (16) est destinée à venir pincer le feuillet (2a) (Cf. figures 17 et 24), tout en étant reliée au cordage artificiel (7). Les mors (16a, 16b) de la pince (16) sont tous deux ajourés pour permettre à une aiguille (18), qui est agencée à l'intérieur et le long de la deuxième tige de manipulation (14), de se déplacer pour venir percer et traverser l'épaisseur du feuillet (2a) de la valve (2) (Cf. figures 25).

L'aiguille (18) permet de fixer le cordage artificiel (7) au feuillet (2a) de la valve mitrale (2). Pour ce faire, l'aiguille (18), après avoir traversé le feuillet (2a) peut saisir le cordage artificiel (7) maintenu par le clapet (17), et être retirée pour faire traverser le cordage artificiel (7) au travers dudit feuillet (2a) pour sa fixation, notamment par nouage. L'aiguille (18) présente donc des moyens pour saisir le cordage artificiel (7), par exemple sous la forme d'une échancrure inclinée formant un crochet à l'extrémité de l'aiguille (18).

Selon une autre technique, illustrée aux figures 18 à 20 et 26 à 28, l'aiguille (18) présente intérieurement une suture (19) à mémoire de forme, apte à être extraite pour traverser l'épaisseur du feuillet (2a), et former une boucle, traversant de nouveau l'épaisseur du feuillet (2a), pour attacher le cordage artificiel (7) audit feuillet (2a).

Dans cette dernière configuration, et en référence aux figures 19-20 et 27-28, le cordage artificiel (7) est fixé au pilier (8) par l'intermédiaire d'une agrafe (9), et au feuillet (2a) par l'intermédiaire d'une suture (19) à mémoire de forme, formant une boucle.

Les caractéristiques du dispositif (1) selon l'invention apportent de nombreux avantages par rapport aux solutions existantes. Le fait de passer par la voie transfémorale permet de réduire la durée et le caractère invasif de l'opération. À noter que les agrafes (9), et les pinces (16) utilisées sont radio-opaques, ou bien comprennent des marqueurs radio-opaques et/ou échographiques permettant au chirurgien d'effectuer un contrôle en 2D ou en 3D de leur position, au fur et à mesure de l'opération. De manière avantageuse, les agrafes (9) utilisées sont gainées de textile pour faciliter leur réhabilitation par les tissus humains.

## Revendications

1. Dispositif (1) de réparation d'un cordage de la valve mitrale (2) d'un cœur (4) par voie transfémorale, et destiné à être positionné dans un introducteur étanche disposé dans une veine fémorale (accès percutané transfémoral) pour pénétrer dans l'oreillette gauche (3) du cœur (4) en passant par sa paroi septale(trans-septal), le dispositif comprenant:
- un premier ensemble (6) apte à coopérer avec une poignée assujettie à des moyens de commande pour l'actionnement de l'ensemble pour la mise en place et la fixation d'un cordage artificiel (7) sur le pilier (8) d'un cordage rompu, ledit ensemble est agencé à l'extrémité d'une tige de manipulation (5) et comprend une agrafe élastique (9) reliée de manière pivotante et amovible à l'extrémité de la tige (5) de sorte à passer, sous l'action des moyens de commande, d'une position repliée le long de la tige (5) à une position basculée angulairement par rapport à la tige (5), pour se placer perpendiculairement face au pilier où le cordage doit être remplacé, le premier ensemble (6) comprend des moyens d'ouverture de l'agrafe (9) pour, sous l'action des moyens de commande, agrafer le pilier (8) du cordage rompu, le premier ensemble (6) comprend des moyens de déverrouillage permettant, sous l'action des moyens de commande, de libérer l'agrafe (9), ladite agrafe (9) étant reliée à un cordage artificiel (7) qui remonte dans la tige (5) ;
- un deuxième ensemble (13) apte à coopérer avec une poignée assujettie à des moyens de commande pour l'actionnement de l'ensemble pour la mise en place et la fixation du cordage artificiel (7) à un feuillet (2a) de la valve (2), le deuxième ensemble (13) est agencé à l'extrémité d'une tige de manipulation (14) et comprend des moyens de fixation (9, 16) destinés à coulisser le long du cordage artificiel (7) et reliés de manière pivotante à l'extrémité de la tige (14) de sorte à passer, sous l'action des moyens de commande, d'une position repliée le long de la tige (14) à une position basculée angulairement par rapport à la tige (14), pour se placer parallèlement au feuillet mitral, les moyens de fixation (9, 16) étant aptes, sous l'action des moyens de commande, à fixer le cordage artificiel (7) audit feuillet (2a).

2. Dispositif (1) selon la revendication 1, ***caractérisé* en ce que** les moyens de fixation (9, 16) comprennent une deuxième agrafe élastique (9), ouvrable, repositionnable et amovible,
des moyens d'ouverture de l'agrafe (9), et des moyens de déverrouillage de l'agrafe (9) similaires à ceux du premier ensemble (6), l'agrafe (9) du deuxième ensemble (13) étant destinée à s'ancrer sur le feuillet (2a) pour y fixer le cordage artificiel (7).

3. Dispositif (1) selon la revendication 2, ***caractérisé* en ce que** le cordage artificiel (7) est cranté, et l'agrafe (9) du deuxième ensemble (13) comprend des moyens pour coulisser sur le cordage artificiel (7) cranté uniquement en direction de l'agrafe (9) du premier ensemble (6).

4. Dispositif (1) selon la revendication 2, ***caractérisé* en ce que** l'agrafe (9) du deuxième ensemble (13) comprend un fil (15) destiné à former une boucle de fixation autour du cordage artificiel (7), le cordage artificiel (7) étant apte à être noué autour de ladite boucle.

5. Dispositif (1) selon la revendication 2, ***caractérisé* en ce que** le deuxième ensemble (13) comprend une pince stabilisatrice reliée de manière pivotante à l'extrémité de la tige (14) de sorte à passer, sous l'action des moyens de commande, d'une position repliée le long de la tige (14) à une position basculée angulairement à la tige (14), pour se trouver parallèle au plan du feuillet et d'une position ouverte à une position fermée pour pincer et stabiliser le feuillet (2a) en vue de l'agrafage.

6. Dispositif (1) selon la revendication 1, ***caractérisé* en ce que** les moyens de fixation (9, 16) comprennent une pince (16) reliée de manière pivotante à l'extrémité de la tige (14) de sorte à passer, sous l'action des moyens de commande, d'une position repliée le long de la tige (14) à une position basculée angulairement à la tige (14), pour se trouver parallèle au plan du tissu du feuillet et d'une position ouverte à une position fermée pour pincer le feuillet (2a), le deuxième ensemble (13) comprenant une aiguille (18) mobile pour venir, sous l'action des moyens de commande, percer le feuillet (2a) au travers d'une ouverture que présente la pince (16) et fixer le cordage artificiel (7) au feuillet (2a).

7. Dispositif (1) selon la revendication 6, ***caractérisé* en ce que** l'aiguille (18) comprend intérieurement une suture (19) à mémoire de forme apte à être extraite pour former une boucle pour fixer le cordage artificiel (7) au feuillet (2a).

8. Dispositif (1) selon la revendication 6, ***caractérisé* en ce que** l'aiguille (18) comprend des moyens pour saisir le cordage artificiel (7) après avoir percé le feuillet (2a).

9. Dispositif (1) selon la revendication 1, ***caractérisé* en ce que** l'agrafe (9) comprend deux branches élastiques (9a) rapprochées l'une de l'autre avec des parties d'extrémités en crochet (9b), les moyens d'ouverture/déverrouillage comprennent deux bras (12) disposés entre les branches élastiques (9a) de l'agrafe (9) et articulés autour d'un pivot (11) de sorte à passer, sous l'action des moyens de commande, d'une position rapprochée l'un de l'autre à une position écartée en forçant l'écartement des branches de l'agrafe (9) pour provoquer son ouverture, les deux bras (12) étant escamotables pour permettre la libération de l'agrafe (9) amovible.

10. Dispositif (1) selon la revendication 6, ***caractérisé* en ce que** la pince (16) comprend un mors supérieur (16b) et un mors inférieur (16a), ajourés pour le passage de l'aiguille (18), le mors inférieur (16a) comprend un clapet (17) destiné à adopter une position fermée pour maintenir le cordage artificiel (7), tout en autorisant le coulissement de la pince (16) autour et le long dudit cordage artificiel (7), et une position ouverte de libération du cordage artificiel (7).

## Patentansprüche

1. Vorrichtung (1) zur Wiederherstellung eines Sehnenfadens der Mitralklappe (2) eines Herzens (4) auf transfemoralem Weg, und dazu bestimmt, in eine dichte Einführungsvorrichtung eingesetzt zu werden, die in einer Femoralvene positioniert ist (transfemoraler, perkutaner Zugang) um in den linken Vorhof (3) des Herzens (4) einzudringen, durch seine Septalwand hindurch (transseptal), die Vorrichtung umfasst dabei:
- eine erste Baugruppe (6), die mit einem Griff zusammenarbeiten kann, der über Steuermittel bedient wird, zur Betätigung der Baugruppe zum Einsetzen und zur Befestigung eines künstlicher Sehnenfadens (7) an der Aufhängung (8) eines gerissenen Sehnenfadens, diese Baugruppe ist an einer Manipulatorstange (5) angeordnet und umfasst eine elastische Klammer (9), die kippbar und abnehmbar mit dem Endstück der Stange (5) verbunden sind, so dass sie unter der Wirkung der Steuermittel aus einer entlang der Stange (5) eingeklappten Position in eine zur Stange (5) abgewinkelte Position geklappt werden kann, um senkrecht zur Aufhängung positioniert zu werden, wo der Sehnenfaden ersetzt werden soll, die erste Baugruppe (6) umfasst Mittel zum Öffnen der Klammer (9), um unter der Wirkung der Steuermittel die Aufhängung (8) des gerissenen Sehnenfadens anzuklammern, die erste Baugruppe (6) umfasst Entriegelungsmitteln, mit denen unter der Wirkung der Steuermittel, die Klammer (9) freigegeben werden kann, diese Klammer (9) ist mit einem künstlichen Sehnenfaden (7) verbunden, der in der Stange (5) nach oben führt;
- eine zweite Baugruppe (13), die die mit einem Griff zusammenarbeiten kann, der über Steuermittel verfügt, zur Betätigung der Baugruppe zum Einsetzen und zur Befestigung des künstlichen Sehnenfadens (7) an einem Segel (2a) der Klappe (2), die zweite Baugruppe (13) ist am Ende einer Manipulatorstange (14) angeordnet und umfasst Befestigungsmittel (9, 16), die entlang des künstlichen Sehnenfadens (7) gleiten können, und kippbar mit dem Endstück der Stange (14) verbunden sind, so dass sie unter der Wirkung des Steuerungsmittels aus einer entlang der Stange (14) eingeklappten Position in eine zur Stange abgewinkelte Position geklappt werden können, um sich parallel zum Mitralsegel zu positionieren, die Befestigungsmittel (9, 16) sind in der Lage, unter der Wirkung der Steuermittel den künstlichen Sehnenfaden (7) mit diesem Segel (2a) zu verbinden.

2. Vorrichtung (1) nach Anspruch 1, ***dadurch gekennzeichnet, dass*** die Befestigungsmittel (9, 16) eine zweite elastische Klammer (9) enthalten, die geöffnet, neu positioniert und weggenommen werden kann, Mittel zum Öffnen der Klammer (9) und Mittel zum Entriegeln der Klammer, (9) ähnlich denen der ersten Baugruppe (6), die Klammer (9) der zweiten Baugruppe (13) ist dabei dazu bestimmt. sich am Segel (2a) zu verankern, um dort den künstlichen Sehnenfaden (7) zu befestigen.

3. Vorrichtung (1) nach Anspruch 2, ***dadurch gekennzeichnet, dass*** der künstliche Sehnenfaden (7) gezahnt ist und die Klammer (9) der zweiten Baugruppe (13) Mittel umfasst, um den gezahnten künstlichen Sehnenfaden (7) ausschließlich in Richtung der Klammer (9) der ersten Baugruppe (6) gleiten zu lassen.

4. Vorrichtung (1) nach Anspruch 2, ***dadurch gekennzeichnet, dass*** die Klammer (9) der zweiten Baugruppe (13) einen Faden (15) umfasst, dazu vorgesehen, eine Befestigungsschlaufe um den künstlichen Sehnenfaden (7) zu bilden, der künstliche Sehnenfaden (7) kann dabei um diese Schlaufe geknotet werden.

5. Vorrichtung (1) nach Anspruch 2, ***dadurch gekennzeichnet, dass*** die zweite Baugruppe (13) eine Stabilisierzange umfasst, die schwenkbar mit dem Endstück der Stange (14) verbunden ist, so dass sie, unter der Wirkung der Steuermittel aus einer entlang der Stange (14) eingeklappten Position in eine zur Stange (14) abgewinkelte Position geklappt werden kann, um parallel zur Segelebene zu liegen und aus einer offenen Position, in eine geschlossene Position, um das Segel (2a) im Hinblick auf das Klammern zu ergreifen und zu stabilisieren.

6. Vorrichtung (1) nach Anspruch 1, ***dadurch gekennzeichnet, dass*** die Befestigungsmittel (9, 16) eine Zange (16) umfassen, die schwenkbar mit dem Endstück der Stange (14) verbunden ist, so dass sie, unter der Wirkung der Steuermittel aus einer entlang der Stange (14) eingeklappten Position in eine zur Stange (14) abgewinkelte Position geklappt werden kann, um parallel zur Ebene des Segelgewebes zu liegen und aus einer offenen Position, in eine geschlossene Position, um das Segel (2a) zu ergreifen, die zweite Baugruppe (13) umfasst eine bewegliche Nadel (18), um unter der Wirkung des Steuerungsmittels das Segel (2a) durch eine in der Zange (16) vorgesehene Öffnung zu durchstechen, und den künstlichen Sehnenfaden (7) am Segel (2a) zu befestigen.

7. Vorrichtung (1) nach Anspruch 6, ***dadurch gekennzeichnet, dass*** die Nadel (18) innen ein Memory- Nahtmaterial (19) enthält, dessen Form es ermöglicht, es zu extrahieren um eine Schlaufe zu bilden, um den künstlichen Sehnenfaden (7) am Segel (2a) zu befestigen.

8. Vorrichtung (1) nach Anspruch 6, **dadurch gekennzeichnet, dass** die Nadel (18) Mittel umfasst, den künstlichen Sehnenfaden (7) nach dem Durchstechen des Segels (2a) zu ergreifen.

9. Vorrichtung (1) nach Anspruch 1, ***dadurch gekennzeichnet, dass*** die Klammer (9) zwei elastische Äste (9a) umfasst, die zueinander gebogen werden, mit hakenförmigen Endstücken (9b), die Mittel zum Öffnen/ Entriegeln umfassen zwei Arme (12), angeordnet zwischen den elastischen Ästen (9a) der Klammer (9) und beweglich um einen Zapfen (11) herum angeordnet, so dass sie unter der Wirkung der Steuermittel, aus einer aufeinander zu geklappten Position in eine gespreizte Position gebracht werden können, wobei die Spreizung der Klammer (9)- Äste erzwungen wird, damit sie sich öffnet, die beiden Arme sind (12) einschiebbar, so dass die bewegliche Klammer (9) freigegeben werden kann.

10. Vorrichtung (1) nach Anspruch 6, ***dadurch gekennzeichnet, dass*** die Zange (16) eine obere Backe (16b) und eine untere Backe (16a) umfasst, durchlöchert zur Passage der Nadel (18), die untere Backe (16a) umfasst eine Klappe (17), die eine geschlossene Position einnehmen soll, um den künstlichen Sehnenfaden (7) zu halten, dabei aber das Gleiten der Zange (16) um und entlang des erwähnten künstlichen Sehnenfadens (7) zu erlauben, sowie eine offene Position, in der der künstliche Sehnenfaden (7) freigegeben wird.

## Claims

1. Device (1) for repair of a mitral valve (2) cord of a heart (4) by transfemoral approach, and intended to be positioned in a sealed inserter arranged in a femoral vein (transfemoral percutaneous access) in order to go into a left atrium (3) of the heart (4) by going through a septal wall thereof (transseptal), the device comprises:
- a first assembly (6) engageable with a handle under control of control means for actuation of an assembly for placement and attachment of an artificial cord (7) on a papillary muscle (8) of a ruptured elongated cord, said assembly is arranged at an end of a manipulation rod (5) and comprises an elastic staple (9) connected pivotably and removably at the end of the rod (5) so as to pass, under the action of the control means, from a position folded back along the rod (5) to a position swung angularly relative to the rod (5) for coming into place perpendicularly in front of the papillary muscle where the cord must be replaced, the first assembly (6) comprises means for opening the staple (9) in order to, under the action of the control means, staple the papillary muscle (8) of the ruptured cord, the first assembly (6) comprises means for unlocking with which, under the action of the control means, to release the staple (9), said staple (9) being connected to an artificial cord (7) which goes back in the rod (5);
- a second assembly (13) engageable with a handle under the control of control means for actuation of the assembly for placement and attachment of the artificial cord (7) to a leaflet (2a) of the valve (2), the second assembly (13) is arranged at an end of a manipulation rod (14) and comprises attachment means (9, 16) intended to slide along the artificial cord (7) and pivotably connected to the end of the rod (14) so as to pass, under action of the control means, from a position folded back along the rod (14) to a position swung angularly relative to the rod (14) for placing parallel to the mitral leaflet, where the means of attachment (9, 16) are able, under the action of the command means, to attach the artificial cord (7) to said leaflet (2a).

2. Device (1) according to claim 1, **characterized in that** the means of attachment (9, 16) comprise a second openable, repositionable and removable elastic staple (9), means for opening the staple (9), and means for unlocking the staple (9) similar to those from the first assembly (6), where the staple (9) from the second assembly (13) is intended to anchor itself in the leaflet (2a) for attaching the artificial cord there (7).

3. Device (1) according to claim 2, **characterized in that** the artificial cord (7) is notched, and the staple (9) from the second assembly (13) comprises means for sliding on the notched artificial cord (7) only in the direction of the staple (9) from the first assembly (6).

4. Device (1) according to claim 2, **characterized in that** the staple (9) from the second assembly (13) comprises a thread (15) intended to form an attachment loop around the artificial cord (7), where the artificial cord (7) is knottable around said loop.

5. Device (1) according to claim 2, **characterized in that** the second assembly (13) comprises a stabilizing clip connected pivotably to the end of the rod (14) so as to pass, under the action of the control means, from a position folded back along the rod (14) to a position swung angularly to the rod (14) to come parallel to the plane of the leaflet, and from an open position to a closed position for clamping and stabilizing the leaflet (2a) in order for stapling.

6. Device (1) according to claim 1, **characterized in that** the attachment means (9, 16) comprise a clip (16) connected pivotably to the end of the rod (14) so as to pass, under the action of the control means, from a position folded back along the rod (14) to a position swung angularly to the rod (14) to come parallel to the plane of a tissue of the leaflet, and from an open position to a closed position for clamping the leaflet (2a), where the second assembly (13) comprises a mobile needle (18) that, under the action of the control means, pierces the leaflet (2a) through an opening in the clip (16) and attaches the artificial cord (7) to the leaflet (2a).

7. Device (1) according to claim 6, **characterized in that** the needle (18) comprises inside thereof a shape memory suture (19) that can be extracted to form a loop for attaching the artificial cord (7) to the leaflet (2a).

8. Device (1) according to claim 6, **characterized in that** the needle (18) comprises means for grasping the artificial cord (7) after having pierced the leaflet (2a).

9. Device (1) according to claim 1, **characterized in that** the staple (9) comprises two elastic branches (9a) brought towards each other with hooked end parts (9b), the opening/unlocking means comprise two arms (12) arranged between the elastic branches (9a) of the staple (9) and articulated around a pivot (11) so as to pass, under the action of the control means, from a position brought close to each other to a separated position by forcing the separation of the branches of the staple (9) to cause opening thereof, since the two arms (12) are retractable to allow the release of the removable staple (9).

10. Device (1) according to claim 6, **characterized in that** the clip (16) comprises an upper jaw (16b) and a lower jaw (16a), with openings for the passage of the needle (18), the lower jaw (16a) comprises a flap (17) intended to adopt a closed position for holding the artificial cord (7) while allowing the sliding of the clip (16) around and along said artificial cord (7), and an open position for release of the artificial cord (7).
